(19) Europäisches Patentamt
European Patent Office
Office européen des brevets

(11) Numéro de publication: **0 046 128**
**B1**

# (12) FASCICULE DE BREVET EUROPEEN

(45) Date de publication du fascicule du brevet: **04.04.84**

(21) Numéro de dépôt: **81420120.8**

(22) Date de dépôt: **31.07.81**

(51) Int. Cl.³: **C 07 C 69/14**, C 07 C 67/293, C 07 C 69/78, C 07 C 53/08, C 07 C 47/06

## (54) Procédé d'hydrocarbonylation et/ou de carbonylation de carboxylates d'alkyles.

(30) Priorité: **07.08.80 FR 8017703**

(43) Date de publication de la demande: **17.02.82 Bulletin 82/7**

(45) Mention de la délivrance du brevet: **04.04.84 Bulletin 84/14**

(84) Etats contractants désignés: **AT BE CH DE FR GB IT LI LU NL SE**

(56) Documents cités:
**DE - A - 2 831 354**
**FR - A - 2 359 811**

(73) Titulaire: **RHONE-POULENC CHIMIE DE BASE, 25, quai Paul Doumer, F-92408 Courbevoie (FR)**

(72) Inventeur: **Gauthier-Lafaye, Jean, 21, rue Duguesclin, F-69006 Lyon (FR)**
Inventeur: **Perron, Robert, La Pecolière, F-69390 Charly (FR)**

(74) Mandataire: **Varniere-Grange, Monique et al, RHONE-POULENC RECHERCHES Centre de Recherches de Saint-Fons Service Brevets B.P. 62, F-69190 Saint-Fons (FR)**

**EP 0 046 128 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

# Procédé d'hydrocarbonylation et/ou de carbonylation de carboxylates d'alkyles

La présente invention a pour objet un procédé d'hydrocarbonylation et/ou de carbonylation de carboxylates d'alkyles, en particulier de carboxylates de méthyle et d'acétates d'alkyles en présence d'eau. Le procédé selon la présente invention peut être représenté par le schéma réactionnel ci-après:

$$R—CO—O—R' + CO + H_2 \longrightarrow R'—CO—O—CH_2—R'$$

$$R—CO—O—CH_2—R' \quad R'—CH_2—OH \quad R'—CHO \qquad (I)$$

$$R—CO—OH \quad R'—CO—OH$$

dans lequel R représente un radical alkyle linéaire ou ramifié ayant de 1 à 16 atomes de carbone, un radical cycloalkyle ayant de 3 à 6 atomes de carbone, un radical phényle ($C_6H_5$-), un radical $C_6H_5-C_xH_{2x}-$ ou un radical $C_xH_{2x+1}-C_6H_4-$, x étant un entier compris entre 1 et 6 ($1 \leq x \leq 6$), R' représente un radical alkyle linéaire ou ramifié ayant de 1 à 5 atomes de carbone, ou un radical $C_6H_5-C_xH_{2x}-$, x ayant la signification précédente, R et R' pouvant par ailleurs être identiques.

Le présent procédé trouve une application particulièrement intéressante dans la préparation d'un ou plusieurs composés choisis parmi l'acétaldéhyde, l'acide acétique, l'éthanol et l'acétate d'éthyle à partir de carboxylates de méthyle et notamment, à partir d'acétate de méthyle.

Des auteurs (Cf. Journal of the American Chemical Society, 100 : 19, 1978, p. 6238–6239) ont montré qu'il est possible de préparer, en particulier, l'acétate d'éthyle par hydrocarbonylation de l'acétate de méthyle en présence simultanée de ruthénium, d'un promoteur iodé, d'un donneur de proton (qui est soit HI engagé initialement à la réaction ou formé in situ à partir de $CH_3I$, soit un acide carboxylique).

Toutefois, le développement à l'échelle industrielle d'une telle technique, dont l'intérêt de principe n'est pas contesté, est largement compromis par la faible activité du systéme catalytique mis en oeuvre.

Récemment il a été proposé (Cf. demande de brevet francais n° 78/20843 et demande de brevet allemand n° 2831354) d'effecteur cette réaction en présence d'un sel de cobalt et d'iode. Dependant les hautes pressions nécessaires pour que le système catalytique développe une activité acceptable ne laissent guère entrevoir de possibilité de développement industriel d'un tel procédé.

Parallélement à cela, un examen de la littérature spécialisée montre que de nombreuses tentatives d'hydrocarbonylation du méthanol pour obtenir sélectivement soit l'acétaldehyde, soit l'éthanol n'ont pas débouché sur des solutions satisfaisantes. Un aperçu des diverses techniques proposées à cet effet peut être trouvé, par exemple, dans la partie introductive du brevet américain n° 4 133 966.

De cette analyse il ressort clairement qu'il serait souhaitable de disposer d'un procédé efficace permettant, le cas échéant, de préparer des aldéhydes, des acides carboxyliques, des alcools »homologues« et, plus particulièrement, des carboxylates d'alkyles à partir de leurs homologues inférieurs. Par alcool homologue on entend l'alcool $R'-CH_2-OH$ figurant dans le schéma (1) ci-avant, et qui contient donc un atome de carboné de plus que l'alcool ($R'-OH$) dont dèrive l'ester de départ. Une premiére catégorie de carboxylates d'alkyles qui peut être obtenue selon la présente invention est représentée par la formule $R-CO-O-CH_2-R'$ dans le schéma (1) ci-avant. Ce type d'esters renferme un atome de carbone de plus que l'ester de départ et peut donc être considéré comme »homologue supérieur« de l'ester de départ. Pour simplifier l'exposé ci-après on appelera également »esters homolugues« les carboxylates d'alkyles de formule $R'-CO-O-CH_2-R'$ figurant dans le schéma (1) ci-avant.

La demanderesse a maintenant découvert de manière tout à fait inattendue qu'il est possible d'hydrocarbonyler et/ou de carbonyler les carboxylates d'alkyles dans un milieu contenant initialement au moins 1% en volume d'eau, sous une pression totale comprise en 50 et 1000 bars et à une température supérieure à 120°C pour abtenir un ou plusieurs composés choisis parmi les acides carboxyliques, les aldéhydes, les alcools et les carboxylates d'alkyles homologues selon le schéma 1 ci-avant d'une manière extrêmement efficace à condition de conduire la réaction en présence d'hydrogène, de ruthénium, de cobalt, d'au moins un promoteur iodé et de vanadium (ou d'un composé du vanadium).Ainsi il apparaît que l'un des constituants essentiels du système catalytique selon la présente invention est le ruthénium. La forme précise sous laquelle le ruthénium est engagé à la réaction n'est pas fondamentale. Conviennent particulièrement bien à la mise en oeuvre du présent procédé des ruthénium carbonyles tels que

$Ru_3(CO)_{12}$, $[Ru(CO)_3Br_2]_2$, et $Ru(CO)_4I_2$

et plus généralement tout composé du ruthénium susceptible de conduire dans les conditions réactionnelles, à l'apparition in situ de ruthénium carbonyles. A ce titre on peut citer notamment le ruthénium métallique sous forme finement divisée, le tribromure de ruthénium, le triiodure de ruthénium, les carboxylates de ruthénium (en particulier l'acétate de ruthénium) et l'acétylacétonate

de ruthénium.

La quantité de ruthénium à mettre en oeuvre n'est pas critique. La teneur en ruthénium du milieu réactionnel ayant une influence positive sur la vitesse de réaction, sera déterminée en fonction de la vitesse que l'on estimera convenable d'atteindre.

De manière générale une quantité comprise entre 0,5 et 100 milliatomes-grammes de ruthénium par litre de milieu réactionnel (m At-g/l) conduit à des résultats satisfaisants. On opère de préférence avec une teneur en ruthénium comprise entre l et 50 m.At-g/l de ruthénium.

Le second constituant essentiel du système catalytique est le cobalt. N'importe quelle source de cobalt susceptible de réagir avec l'oxyde de carbone dans le milieu de réaction pour donner des complexes carbonyles du cobalt peut être utilisée dans le cadre du présent procédé.

Les sources typiques de cobalt sont par exemple le cobalt métallique finement divisé, des sels inorganiques tel que le nitrate ou le carbonate de cobalt, des sels organiques en particulier des carboxylates. Peuvent également être employés les cobalt-carbonyles ou hydrocarbonyles.

Parmi les dérivés du cobalt convenant pour la mise en oeuvre du procédé selon l'invention, on peut citer le formiate, l'acétate, les halogénures de cobalt, et plus particulièrement l'iodure de cobalt et le dicobaltoctacarbonyle.

La quantité précise de cobalt engagée à la réaction n'est pas fondamentale. En général, on opère avec une quantité de cobalt telle que le rapport atomique Co/Ru soit compris entre 0,01 et 100 ($0,01 \leq Co/Ru \leq 100$). De préférence ce rapport est compris entre 0,1 et 10.

La mise en oeuvre du procédé selon la présente invention nécessite également la présence d'un promoteur iodé. On peut utiliser à cet effet l'iode libre ou combiné.

Une première catégorie de promoteurs iodés convenant à la mise en oeuvre du présent procédé est constituée par les iodures d'alkyle ou d'acyle, de formules respectives R''−I et R''−CO−I, dans lesquelles R'', pouvant être identique ou différent de R', a la signification donnée pour R'. Dans cette catégorie, on préfère utiliser les iodures d'alkyle ayant au maximum 4 atomes de carbone, et plus particulièrement l'iodure de méthyle ou d'éthyle.

Une seconde catégorie de promoteurs iodés utilisables dans le cadre du présent procédé est constituée par les iodures ioniques dont les cations sont choisis parmi les cations des métaux alcalins, les cations des métaux alcalino-terreux et les cations ammonium ou phosphonium quaternaire représentés par les formules I à III ci-après:

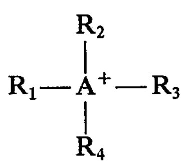

(I)

dans laquelle A représente un atome d'azote ou de phosphore, $R_1$, $R_2$, $R_3$ et $R_4$, pouvant être identiques ou différents représentent l'hydrogène ou, de préférence, des radicaux organiques dont la valence libre est portée par un atome de carbone, deux quelconques de ces divers radicaux pouvant éventuellement former ensemble un radical unique divalent.

Plus spécifiquement $R_1$, $R_2$, $R_3$ et $R_4$ peuvent représenter des radicaux alkyles linéaires ou ramifiés, des radicaux cycloalkyles, aralkyles (par exemple benzyle) ou aryles monocycliques, ayant au plus 16 atomes de carbone, pouvant le cas échéant être substitués par 1 à 3 radicaux alkyles ayant de 1 à 4 atomes de carbone; deux des radicaux $R_1$ à $R_4$ pouvant éventuellement former ensemble un radical unique divalent — alkylène ou alcénylène — comportant 3 6 atomes de carbone (par exemple un radical tétraméthylène ou hexaméthylène) et le cas échéant 1 ou 2 doubles liaisons éthyléniques, ledit radical pouvant porter 1 à 3 substituants alkyles ayant de 1 à 4 atomes de carbone.

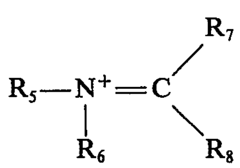

(II)

dans laquelle $R_5$, $R_6$, $R_7$ et $R_8$ identiques ou différents représentent des radicaux alkyles ayant de 1 à 4 atomes de carbone, l'un des radicaux $R_7$ ou $R_8$ pouvant en outre représenter l'hydrogène, $R_7$ et $R_8$ pouvant éventuellement former ensemble un radical unique divalent alkylène comportant de 3 à 6 atomes de carbone, tétraméthylène ou hexaméthylène par exemple; $R_6$ et $R_7$ ou $R_8$ peuvent former ensemble un radical unique divalent — alkylène ou alcénylène — comportant 4 atomes de carbone et le cas échéant 1 ou 2 doubles liaisons éthylèniques, l'atome d'azote étant alors inclus dans un hétérocycle, pour constituer, par exemple, un cation pyridinium.

$$(R_9)_2A^+ - (CH_2)_y - A^+(R_9)_2 \quad \text{with } R_5 \text{ on each } A^+$$ (III)

dans laquelle $R_5$ et $A^+$ ont la signification donnée précédemment, $R_9$ pouvant être identique à $R_5$ représente un radical alkyle ayant de 1 à 4 atomes de carbone ou un radical phényle, et y est un entier compris entre 1 et 10 ($1 \leq y \leq 10$) et de préférence entre 1 et 6 ($1 \leq y \leq 6$). A titre d'exemple d'iodures d'ammonium quaternaire convenant à la mise en oeuvre du présent procédé on peut citer les iodures

de tétraméthylammonium
de triéthylméthylammonium
de tributylméthylammonium,
de triméthyl(n-propyl)ammonium,
de tétraéthylammonium,
de tétrabutylammonium,
de dodécyltriméthylammonium,
de benzyltriméthylammonium,
de benzyldiméthylpropylammonium,
de benzyldiméthyloctylammonium,
de diméthyldiphénylammonium,
de méthyltriphénylammonium,
de N,N-diméthyl-triméthylènammonium,
de N,N-diéthyl-triméthylènammonium,
de N,N-diméthyl-tétraméthylènammonium,
de N,N-diéthyl-tétraméthylènammonium,
de N-méthylpyridinium
de N-éthylpyridinium
de N-méthylpicolinium

A titre d'exemple d'iodures de phosphonium quaternaire convenant également à la mise en oeuvre du présent procédé, on peut citer les iodures

de tétraméthylphosphonium,
d' éthyltriméthylphosphonium,
de triméthylpentylphosphonium,
d' octyltriméthylphosphonium,
de dodécyltriméthylphosphonium,
de triméthylphénylphosphonium,
de diéthyldiméthylphosphonium,
de dicyclohexyldiméthylphosphonium,
de diméthyldiphénylphosphonium,
de cyclohexyltriméthylphosphonium,
de triéthylméthylphosphonium,
de méthyl-tri(isopropyl)phosphonium,
de méthyl-tri(n-propyl)phosphonium,
de méthyl-tri(n-butyl)phosphonium,
de méthyl-tris(méthyl-2-propyl)phosphonium,
de méthyltricyclohexylphosphonium,
de méthyltriphénylphosphonium,
de méthyltribenzylphosphonium,
de méthyl-tris(méthyl-4 phényl)phosphonium,
de méthyltrixylylphosphonium,
de dièthylmèthylphénylphosphonium,
de dibenzylméthylphénylphosphonium,
d' éthyltriphénylphosphonium,
de tétraéthylphosphonium,
d' èthyl-tri(n-propyl)phosphonium,
de triéthylpentylphosphonium,
d' éthyltriphénylphosphonium,
de n-butyl-tri(n-propyl)phosphonium,
de butyltriphénylphosphonium,
de benzyltriphénylphosphonium,
de ($\beta$-phényléthyl)diméthylphénylphosphonium,
de tétraphénylphosphonium,
de triphényl(méthyl-4 phényl)phosphonium

La nature précise du cation ammonium ou phosphonium quaternaire n'est pas fondamentale dans le cadre du présent procédé. Le choix parmi ces composés est davantage orienté par des considérations d'ordre pratique, telles que la solubilité dans le milieu réactionnel, la disponibilité et la commodité d'emploi.

A ce titre, les iodures d'ammonium ou de phosphonium quaternaire représentés soit par la formule (I) dans laquelle l'un quelconque des radicaux $R_1$ à $R_4$ est choise parmi les radicaux alkyles linéaires ayant de 1 à 4 atomes de carbone, soit par les formules (II) ou (III) dans laquelle $R_5$ ou ($R_6$) est également un radical alkyle ayant de l à 4 atomes de carbone, coniennent particulièrement bien.

En outre, parmi les iodures d'ammonium on préfère ceux dont les cations correspondent à la formule (I) dans laquelle tous les radicaux $R_1$ à $R_4$ sont choisis parmi les radicaux alkyles linéaires ayant de 1 à 4 atomes de carbone et dont au moins trois d'entre eux sont identiques.

De même, parmi les iodures de phosphonium quaternaire, on préfère ceux dont les cations correspondent à la formule (I) dans laquelle l'un quelconque des radicaux $R_1$ à $R_4$ représente un radical alkyle linéaire ayant de 1 à 4 atomes de carbone, les trois autres radicaux étant identiques et choisis parmi les radicaux phényle, tolyle o xylyle.

Les iodures des métaux alcalins, en particulier les iodures de lithium, de potassium et de sodium constituent une classe préférée d'iodures ioniques dans le cadre de la présente invention. Les iodures de phosphonium quaternaire et plus particulièrement ceux dont les cations correspondent à la formule (I) ci-avant, dans laquelle l'un des radicaux $R_1$ à $R_4$ est un radical alkyle ayant de 1 à 4 atomes de carbone, les trois autres radicaux étant identiques et choisis parmi les radicaux phényle, tolyle ou xylyle, constituent une autre classe d'iodures ioniques particulièrement efficaces pour la mise en oeuvre de la présente invention.

Bien entendu, l'acide iodhydrique peut être utilisé comme promoteur iodé; des composés iodés tels que $CoI_2$, $RuI_3$ et $Ru(CO)_4I_2$ peuvent également être employés seuls ou, de préference en mélange avec un ou plusieurs promoteur(s) iodé(s) appartenant à l'une ou à l'autre des catégories mentionnées ci-avant.

En général, la quantité de promoteur(s) iodé(s) est telle que le rapport atomique I/Ru soit au moins égal à 0,01; il n'est pas utile de dépasser la valeur de 2000 pour ce rapport. D'une maniére avantageuse ce rapport est compris entre 0,05 et 500.

Selon une variante préférée du présent procédé on utilise simultanément un iodure d'alkyle ou d'acyle (membre de la première catégorie de promoteurs iodés définie ci-avant) et un iodure ionique appartenant à la seconde catégorie de promoteurs iodés précitée.

La demanderesse a constaté que de bons résultats sont obtenus lorsqu'on utilise simultanément un iodure d'alkyle ($R''-I$), et un iodure alcalin.

L'utilisation simultanée d'iodure de méthyle et d'un iodure alcalin s'avère particulièrement avantageuse, dans le cadre du présent procédé.

L'une des caractéristiques essentielles de la présente invention réside dans la mise en oeuvre de vanadium ou d'un composé de vanadium. Bien que le vanadium métallique, finement divisé, puisse être utilisé la demanderesse préconise l'emploi des composés du vanadium dans lesquels le vanadium est au degré d'oxydation 4 ou 5, c'est à dire d'au moins un composé choisi dans le groupe constitué par les oxydes, les halogénures, les oxyhalogénures de vanadium (IV) ou de vanadium (V) et le bis acétylacétonate de vanadyle. A tritre d'exemples de composés du vanadium convenant à la mise en oeuvre du present procédé on peut citer: $VCl_4$, $VOCl_2$, $VOBr_2$, $VO(C_5H_7O_2)_2$, $V_2O_5$, $VOCl_3$ et $VOBr_3$.

Parmi les composés du vanadium (IV), le bisacétylacétonate de vanadyle $VO(C_5H_7O_2)_2$ s'avère particulièrement efficace.

La quantité de vanadium (ou de composé du vanadium) engagée à la réaction est en général telle que le rapport atomique V/Ru soit compris entre 0,5 et 500 ($0{,}5 \leq V/Ru \leq 500$). De préférence ce rapport est compris entre 1 et 200.

Selon la présente invention on fait donc réagir un mélange renfermant du monoxyde de carbone et de l'hydrogène sur un carboxylate d'alkyle en présence d'eau, l'eau représentant au moins 1% en volume du milieu réactionnel initial et du système catalytique défini ci-avant. L'eau peut atteindre 25% en volume du milieu réactionnel initial. La réaction est avantageusement conduite en phase liquide. On opère sous une pression totale comprise entre 50 et 1000 bars, et pour une bonne mise en oeuvre de l'invention, une pression totale de 80 à 350 bars est préconisée. Le rapport molaire du monoxyde de carbone à l'hydrogéne peut varier dans de larges limites.

Lorsqu'on souhaite favoriser la carbonylation de la matieère de départ, (production de l'acide carboxylique $R'-CO-OH$) on opère avec un mélange comprenant une proportion prépondérante de monoxyde de carbone et une faible proportion d'hydrogène; en général un rapport molaire $CO/H_2$ supérieur à 5 conduit à des résultats satisfaisants dans ce cas.

Lorsqu'on souhaite favoriser l'hydrocarbonylation de la matière de départ (production de $R'-CHO$, $R'-CH_2OH$, $R-CO-O-CH_2-R'$ et $R'-CO-O-CH_2-R'$), on opère avec un mélange renfermant du monoxyde de carbone et de l'hydrogène dans un rapport molaire $CO/H_2$ compris entre 1/10 et 10/1, et de préférence entre 1/5 et 5/1.

Dans tous les cas, on met en oeuvre du monoxyde de carbone et de l'hydrogène sensiblement purs, tels qu'ils se présentent dans le commerce. Toutefois la présence d'impuretés telles que, par exemple

du dioxyde de carbone, de l'oxygène, du méthane et de l'azote n'est pas nuisible.

La température de réaction est supérieure à 120°C. Toutefois il n'est pas utile de dépasser la température de 300°C. De bons résultats sont obtenus dans la gamme de températures allant de 160 à 250°C.

Comme indiqué dans le schéma (1) le produit de départ est un carboxylate d'alkyle de formule $R-CO-O-R'$ dans laquelle R représente un radical alkyle linéaire ou ramifié ayant de 1 à 16 atomes de carbone, un radical cycloalkyle ayant de 3 à 6 atomes de carbone, un radical phényle ($C_6H_5-$), un radical $C_6H_5-C_xH_{2x}-$ ou un radical $C_xH_{2x+1}-C_6H_5-$, x étant un entier compris entre 1 et 6 ($1 \leq x \leq 6$) et R' représente un radical alkyle linéaire ou ramifié ayant de 1 à 5 atomes de carbone, R et R' pouvant par ailleurs être identiques. De préference R' est un radical méthyle. R est avantageusement un radical alkyle ayant au plus 4 atomes de carbone ou un radical $C_6H_5-CH_2-$.

Les acétates et les benzoates d'alkyles et plus particulièrement l'acétate et le benzoate de méthyle s'avèrent être des matières de départ particulièrement appropriées dans le cadre de la présente invention.

Bien entendu, le carboxylate d'alkyle (matière de départ), peut être formé in-situ à partir de l'acide carboxylique et de l'alcool correspondant, de formules respectives RCOOH et R'OH.

La demanderesse a constaté que de bons résultats sont obtenus lorsque le milieu réactionnel renferme, en outre, initialement, un acide carboxylique de formule R'''COOH dans laquelle R''' a la signification donnée pour R, R''' et R pouvant être identiques ou différents. Le milieu réactionnel initial peut contenir jusqu'à 90% en volume d'acide carboxylique (R'''COOH).

Selon une variante avantageuse du présent procédé le milieu réactionnel initial renferme de 1 à 20% en volume d'eau et de 5 à 50% en volume d'acide carboxylique.

Lorsque le milieu réactionnel initial renferme un acide carboxylique (R'''COOH), distinct de l'acide RCOOH dont est issu le carboxylate d'alkyle de départ, on not parfois la présence, parmi les produits de réaction, de l'ester de formule:

$R'''COOCH_2R'$, dans laquelle R' a la signification précédente.

Lorsqu'on souhaite opérer en présence initiale d'un acide carboxylique (R'''COOH), on fait appel, de préférence à l'acide acétique, propionique, butyrique, benzoïque ou toluique.

Comme indiqué précédement, le présent procédé trouve une application particulièrement intéressante dans la préparation d'un ou plusieurs composés choisis parmi l'acétaldéhyde, l'acide acétique, l'éthanol et l'acétate d'éthyle à partir de carboxylates de méthyle et notamment à partir d'acétate de méthyle.

Pour autant qu'on puisse le déterminer, et sans que cela limite l'invention, le procédé, décrit dans le présent mémoire, qui conduit à la formation des produits principaux rappelés ci-avant, peut être orienté vers la production préférentielle de l'une ou l'autre des classes de produits en cause.

Ainsi, une diminution de la température de réaction et/ou de la proportion d'hydrogène dans le mélange $CO/H_2$ est susceptible de favoriser la production d'acide acétique. (Bien entendu dans le cas où la matière de départ est l'acétate de méthyle, une partie de l'acide acétique formé provient de la réaction d'hydrolyse de la matière de départ). Par contre, une augmentation de la température de réaction et/ou de la proportion d'hydrogène dans le mélange $CO/H_2$ et, le cas échéant, la présence dans le milieu réactionnel d'une proportion accrue de ruthénium semblent orienter la réaction vers la formation préférentielle de l'acétate d'éthyle, voire de l'éthanol.

Une augmentation de la température et/ou de la proportion d'hydrogène dans le mélange $CO/H_2$, associée à une diminution de la durée de réaction tendrait à favoriser la production d'acétaldéhyde.

Ainsi il est possible d'obtenir l'acétaldéhyde libre, c'est à dire sensiblement non transformé en diméthylacétal, contrairement à ce qui se produit de manière plus ou moins prononcée lorsqu'on cherche à obtenir ce produit au départ du méthanol libre. L'homme de l'art appréciera le fait que la récupération de l'acétaldéhyde du milieu réactionnel est facilitée, notamment lorsqu'on opère en présence d'un acide carboxylique lourd tel que l'acide benzoique et/ou lorsqu'on choisit comme matière de départ un carboxylate de méthyle lourd (par exemple, le benzoate de méthyle).

En fin de réaction, les produits obtenus peuvent être aisément séparés, par distallation fractionnée du mélange résultant, par exemple.

Les exemples ci-après illustrent l'invention, sand toutefois en limiter le domaine ou l'esprit.

Le mode opératoire utilisé est le suivant

Dans un autoclave en acier inoxydable Z-8 CNDT 17-12 (norme AFNOR) de 250 ml de capacité on charge le carboxylate d'alkyle (matière de départ), le système catalytique, de l'eau distillée et le cas échéant un acide carboxylique. Après fermeture de l'autoclave, on établit une pression de 140 bars (sauf indications contraires) à l'aide d'un mélange de monoxyde de carbone et d'hydrogène dans un rapport molaire fixé et indiqué pour chaque exemple ci-après.

L'agitation par un système de va et vient est mise en route et l'autoclave est alors porté à la température choisie, en 25 minutes environ.

La pression dans l'autoclave s'élève alors et est maintenue sensiblement à la valeur indiquée dans

chacun des exemples ci-après par des recharges successives du mélange $CO/H_2$ initial. Lorsque la durée de réaction fixée pour chaque exemple, à la température indiquée, est atteinte, le chauffage et l'agitation sont arrêtés. L'autoclave est alors refroidi et dégazé. Le mélange réactionnel résultant est analysé par chromotographie gazeuse, après dilution.

Les conventions suivantes sont utilisées

Les résultats obtenus sont indiqués en moles de produits (en principe: acétaldéhyde, éthanol, acétate d'éthyle et acide acétique) obtenues par heure et par litre de mélange réactionnel. Pour chaque produit on utilise la notation M/hxl.

Les résultats relatifs à l'acide acétique n'incluent ni la quantité d'acide acétique susceptible d'être chargée initialement, ni la quantité qui s'est formée par hydrolyse de l'acétate de méthyle (matière de départ).

On a indiqué également par RT pour un produit donné la sélectivité de ce produit par rapport à l'ensemble des produits dont la liste figure ci-avant.

Le taux de transformation noté TT dans ce qui suit est défini comme étant le rapport du nombre total de moles de produits dont la liste est dressée précédemment au nombre de moles d'acétate de méthyle chargées, diminué du nombre de moles (de cette matière de départ) hydrolysées.

(La partie de matière de départ hydrolysée au cours d'un essai peut être déterminée par dosage du méthanol contenu dans le produit liquide obtenu en fin d'essai).

Exemples 1 à 11

Dans l'autoclave et selon le mode opératoire décrit ci-avant, on réalise une série d'essais sur une charge renfermant 80 ml d'acétate de méthyle (1000 mMol), 20 ml d'acide acétique (350 mMol), 3 ml d'eau (170 mMol), du cobalt, du trituthéniumdodécacarbonyle, de l'acétylacétonate de vanadyle, de l'iodure de méthyle et/ou de l'iodure de sodium. Les conditions particulières figurent dans le tableau I(a) ci-après dans lequel $P_T$ désigne la pression totale, T la température de réaction et $Co(OAc)_2$ l'acétate de cobalt tétrahydraté; les résultats obtenus sont portés dans le tableau I(b) ci-après dans lequel AcOH désigne l'acide acétique et MeOH le méthanol. L'essai témoin (a) est réalisé en l'absence de vanadium.

Tableau I (a)

Conditions opératoires

| Ex n° | Ru mAt-g | Cobalt nature | mAt-g | V mAt-g | $CH_3I$ mMol | NaI mMol | $P_T$ bars | $CO/H_2$ | T°C | Durée mn |
|---|---|---|---|---|---|---|---|---|---|---|
| 1 | 1,30 | $Co(OAc)_2$ | 0,22 | 17 | 35 | 0 | 250 | 1/2 | 215 | 20 |
| 2 | 1,30 | $Co(OAc)_2$ | 0,22 | 8,5 | 0 | 30 | 243 | 1/2 | 212 | 20 |
| 3 | 1,30 | $Co_2(CO)_8$ | 0,22 | 16,7 | 3,52 | 12 | 260 | 1/2 | 216 | 40 |
| a | 1,30 | $Co_2(CO)_8$ | 0,22 | 0 | 3,52 | 12 | 260 | 1/2 | 216 | 75 |
| 4 | 1,30 | $CoI_2$ | 0,22 | 16,7 | 3,60 | 0 | 260 | 1/1 | 212 | 40 |
| 5 | 1,30 | $CoI_2$ | 0,22 | 16,7 | 3,61 | 15 | 260 | 1/1 | 214 | 40 |
| 6 | 1,30 | $CoI_2$ | 0,22 | 16,7 | 3,62 | 30 | 260 | 1/1 | 212 | 40 |
| 7 | 1,30 | $CoI_2$ | 0,22 | 4,1 | 3,79 | 15 | 260 | 1/1 | 215 | 40 |
| 8 | 1,30 | $CoI_2$ | 0,22 | 29,7 | 3,67 | 15 | 250 | 1/1 | 215 | 40 |
| 9 | 0,65 | $CoI_2$ | 0,22 | 29,7 | 3,54 | 15 | 250 | 1/1 | 214 | 40 |
| 10 | 1,30 | $CoI_2$ | 2,89 | 29,7 | 0 | 15 | 260 | 1/1 | 214 | 40 |
| 11 | 1,30 | $CoI_2$ | 0,22 | 29,7 | 16,1 | 15 | 260 | 1/1 | 212 | 40 |

Tableau I (b)

Resultats

| n° | Acétaldéhyde | | Ethanol | | Acétate d'éthyle | | Acide acétique | | TT |
|---|---|---|---|---|---|---|---|---|---|
| Ex | M/hxl | RT (%) | M/hxl | RT (%) | M/hxl | RT (%) | M/hxl | RT (%) | (%) |
| 1 | 0,41 | 6 | 1,51 | 22 | 3,56 | 51 | 1,51 | 22 | 23,3 |
| 2 | 0,82 | 16 | 1,40 | 28 | 2,87 | 56 | – | – | 17,4 |
| 3 | 0,32 | 6 | 0,91 | 18 | 3,26 | 65 | 0,52 | 10 | 33,6 |
| a | 0,18 | 9 | 0,32 | 16 | 1,45 | 72 | 0,06 | 3 | 25,1 |
| 4 | – | – | 0,08 | 11 | 0,41 | 58 | 0,22 | 31 | 3,2 |
| 5 | 1,47 | 30 | 0,59 | 12 | 1,91 | 38 | 1,01 | 20 | 34,6 |
| 6 | 1,31 | 30 | 0,29 | 7 | 1,70 | 39 | 1,09 | 25 | 31 |
| 7 | 0,99 | 24 | 0,59 | 14 | 1,69 | 40 | 0,94 | 22 | 28,3 |
| 8 | 1,14 | 26 | 0,29 | 7 | 1,97 | 45 | 0,99 | 23 | 30,7 |
| 9 | 1,40 | 35 | 0,26 | 6 | 1,25 | 31 | 1,12 | 28 | 28,2 |
| 10 | 1,84 | 30 | 0,39 | 6 | 1,83 | 30 | 2,14 | 34 | 44,6 |
| 11 | 1,28 | 30 | 0,28 | 7 | 1,75 | 41 | 0,93 | 22 | 29,7 |

## Exemple 12

Dans l'autoclave et selon le mode opératoire décrit ci-avant, on réalise un essai sur une charge constituée de:

- 90 ml d'acétate de méthyle (1130 mMol),
- 5 ml d'acide acétique ( 87,5 mMol),
- 10 ml d'eau ( 566 mMol),
- 0,24 mAt-g de cobalt sous la forme d'acétate de cobalt,
- 1,31 mAt-g de ruthénium sous la forme d'iodure de ruthénium,
- 29,7 mAt-g de vanadium sous la forme d'acétylacétonate de vanadyle,
- 10 mMol d'iodure de potassium.

En 40 minutes de réaction à 203° C, la pression dans l'autoclave étant maintenue vers 200 bars par des recharges périodiques d'un mélange $CO/H_2$ : 1/1, on observe la formation de:

- 0,07 M/h × l d'acétaldéhyde (RT= 6%)
- 0,34 M/h × l d'éthanol (RT=28%)
- 0,31 M/h × l d'acétate d'éthyle (RT=25%)
- 0,51 M/h × l d'acide acétique (RT=41%)
(TT=7,2%)

## Exemple 13

Dans l'autoclave et selon le mode opératoire décrit ci-avant, on réalise un essai sur une charge constituée constituée de:

- 75 ml d'acétate de méthyle (940 mMol),
- 25 ml d'acide acétique (437 mMol),

- 3 ml d'eau (170 mMol),
- 0,55 mAt-g de cobalt sous la forme d'iodure de cobalt,
- 1,32 mAt-g de ruthénium sous la forme d'acétylacétonate de ruthénium,
- 26 mAt-g de vanadium sous la forme d'acétylacétonate de vanadyle,
- 10,5 mMol d'iodure de méthyle,
- 12 mMol d'iodure de sodium.

En 40 minutes de réaction à 213°C, la pression dans l'autoclave étant maintenue vers 150 bars par des recharges périodiques d'un mélange $CO/H_2$ : 1/2, on observe la formation de:

- 0,22 M/h × l d'acétaldéhyde (RT = 10%)
- 0,21 M/h × l d'éthanol (RT = 9%)
- 0,93 M/h × l d'acétate d'éthyle (RT = 41%)
- 0,93 M/h × l d'acide acétique (RT = 41%)

(TT = 16,3%)

Exemple 14

Dans l'autoclave et selon le mode opératoire décrit ci-avant, on réalise un essai sur une charge constituée de:

- 80 ml d'acétate de méthyle (1000 mMol),
- 20 ml d'acide acétique ( 350 mMol),
- 3 ml d'eau ( 170 mMol),
- 0,22 mAt-g de cobalt sous la forme d'iodure de cobalt,
- 1,32 mAt-g de ruthénium sous la forme d'acétylacétonate de ruthénium,
- 8,36 mAt-g de vanadium sous la forme d'acétylacétonate de vanadyle,
- 30 mMol d'iodure de sodium.

En 20 minutes de réaction à 213°C, la pression dans l'autoclave étant maintenue vers 260 bars par des recharges périodiques d'un mélange $CO/H_2$ : 1/2, on observe la formation de:

- 1,04 M/h × l d'acétaldéhyde (RT = 13%)
- 1,55 M/h × l d'éthanol (RT = 20%)
- 2,97 M/h × l d'acétate d'ethyle (RT = 38%)
- 2,35 M/h × l d'acide acétique (RT = 30%)

(TT = 26,3%).

Exemple 15

Dans l'autoclave et selon le mode opératoire décrit ci-avant, on réalise un essai sur une charge constituée de:

- 60 ml d'acétate de méthyle (750 mMol),
- 40 ml d'acide acétique (700 mMol),
- 5 ml d'eau (280 mMol),
- 0,23 mAt-g de cobalt sous la forme d'iodure de cobalt,
- 1,34 mAt-g de ruthénium sous la forme de chlorure de ruthénium,
- 16,7 mAt-g de vanadium sous forme d'acétylacétonate de vanadyle,
- 5 mMol d'iode,
- 20 mMol d'iodure de lithium.

En 40 minutes de réaction à 211°C, la pression dans l'autoclave étant maintenue vers 205 bars des recharges périodiques d'un mélange $CO/H_2$ : 1/2, on oberve la formation de:

- 0,12 M/h × l d'acétaldéhyde (RT = 5%)
- 0,20 M/h × l d'éthanol (RT = 9%)
- 1,01 M/h × l d'acétate d'éthyle (RT = 45%)
- 0,93 M/h × l d'acide acétique (RT = 41%)

(TT = 20%)

Exemple 16

Dans l'autoclave et selon le mode opératoire décrit ci-avant, on réalise un essai sur une charge constituée de:

- 80 ml de benzoate de méthyle (635 mMol),
- 20 ml d'acide acétique (350 mMol),
- 3 ml d'eau (170 mMol),
- 0,23 mAt-g de cobalt sous la forme d'iodure de cobalt,
- 1,25 mAt-g de ruthénium sous la forme d'acétylacétonate de ruthénium,
- 8,36 mAt-g de vanadium sous la forme d'acétylacétonate de vanadyle,
- 5,25 mMol d'iodure de méthyle,
- 6 mMol d'iodure de méthyltriphénylphosphonium.

En 40 minutes de réaction à 213° C, la pression dans l'autoclave étant maintenue vers 205 bars par des recharges périodiques d'un mélange $CO/H_2$ : 1/2, on observe la formation de:

- 0,48 M/h × l d'acétaldéhyde
- 0,54 M/h × l d'éthanol
- 0,96 M/h × l d'acétate d'éthyle
- 0,20 M/h × l de benzoate d'éthyle

Exemple 17

Dans l'autoclave et selon le mode opératoire décrit ci-avant, on réalise un essai sur une charge constituée de:

- 80 ml d'acétate de méthyle (1000 mMol),
- 20 ml d'acide acétique ( 350 mMol),
- 3 ml d'eau ( 170 mMol),
- 0,22 mAt-g de cobalt sous la forme d'iodure de cobalt,
- 1,30 mAt-g de ruthénium sous la forme de triruthéniumdodécacarbonyle.
- 16,7 mAt-g de vanadium sous la forme d'acétylacétonate de vanadyle,
- 3,53 mMol d'iodure de méthyle,
- 15 mMol d'iodure de tétraéthylammonium.

En 10 minutes de réaction à 214° C, la pression dans l'autoclave étant maintenue vers 260 bars par des recharges périodiques d'un mélange $CO/H_2$ : 1/1, on observe la formation de:

- 6,20 M/h × l d'acétaldéhyde (RT=69%)
- 0,72 M/h × l d'éthanol (RT= 8%)
- 2,01 M/h × l d'acétate d'éthyle (RT=23%)

(TT=14,9%)

Exemple 18

Dans l'autoclave et selon le mode opératoire décrit ci-avant, on réalise un essai sur une charge constituée de:

- 80 ml d'acétate de méthyle (1000 mMol),
- 20 ml d'acide acétique ( 350 mMol),
- 5 ml d'eau ( 280 mMol),
- 0,22 mAt-g de cobalt sous la forme d'acétate tétrahydraté,
- 1,30 mAt-g de ruthénium sous la forme de triruthéniumdodécacarbonyle,
- 8,36 mAt-g de vanadium sous la forme d'acétylacétonate de vanadyle,
- 30 mMol d'iodure de sodium.

En 20 minutes de réaction à 214° C, la pression dans l'autoclave étant maintenue vers 250 bars par des recharges périodiques d'un mélange $CO/H_2$ : 13/1, on observe la formation de:

- 1,15 M/h × l d'acétaldéhyde (RT = 25%)
- 0,18 M/h × l d'éthanol (RT = 4%)
- 0,42 M/h × l d'acétate d'éthyle (RT = 9%)
- 2,93 M/h × l d'acide acétique (RT = 63%)

(TT = 15,5%)

## Revendications

1. Procédé d'hydrocarbonylation et/ou de carbonylation de carboxylates d'alkyles dans un milieu contenant initialement au moins 1% en volume d'eau, sous une pression totale comprise en 50 et 1000 bars et à une température supérieure à 120° C caractérisé en ce que la réaction est conduite en présence d'hydrogène, de ruthénium, de cobalt, d'au moins un promoteur iodé et de vanadium.

2. Procédé selon la revendication 1, caractérisé en ce que le carboxylate d'alkyle à pour formule $R-CO-OR'$ dans laquelle R représente un radical alkyle linéaire ou ramifié ayant de 1 à 16 atomes de carbone, un radical cycloalkyle ayant de 3 à 6 atomes de carbone, un radical phényle ($C_6H_5-$), un radical $C_6H_5-C_xH_{2x}-$ ou un radical $C_xH_{2x+1}-C_6H_4-$, x étant un entier compris entre 1 et 6 ($1 \leq x \leq 6$), R' représente un radical alkyle linéaire ou ramifié ayant de 1 à 5 atomes de carbone, ou un radical $C_6H_5-C_xH_{2x}-$, x ayant la signification précédente, R et R' pouvant par ailleurs être identiques.

3. Procédé selon la revendication 2, caractérisé en ce que R' représente un radical méthyle.

4. Procédé selon la revendication 2 ou 3, caractérisé en ce que R représente un radical alkyle ayant au plus 4 atomes de carbone ou un radical $C_6H_5-CH_2-$.

5. Procédé selon la revendication 1, caractérisé en ce que la quantité de ruthénium est comprise entre 0,5 et 100 mAt-g, et de préférence entre 1 et 50 mAt-g par litre de milieu réactionnel.

6. Procédé selon la revendication 1 ou 5, caractérisé en ce que le rapport atomique Co/Ru est compris entre 0,01 et 100, et de préférence, entre 0,1 et 10.

7. Procédé selon l'une quelconque des revendications précédentes, caractérisé en ce que le promoteur iodé utilisé est un iodure ionique dont le cation est choisi parmi les cations des métaux alcalins, les cations des métaux alcalino-terreux, les cations ammonium quaternaire, et les cations phosphonium quaternaire.

8. Procédé selon l'une quelconque des revendications 1 à 6, caractérisé en ce que le promoteur iodé est un iodure d'alkyle ou d'acyle.

9. Procédé selon l'une quelconque des revendications précédentes, caractérisé en ce que le rapport atomique I/Ru est compris entre 0,01 et 2000, et de préférence entre 0,05 et 500.

10. Procédé selon la revendication 8 ou 9, caractérisé en ce qu'on utilise conjointement un iodure d'alkyle et un iodure ionique dont le cation est choisi parmi les cations des métaux alcalins, les cations des métaux alcalino-terreux, les cations ammonium quaternaire, et les cations phosphonium quaternaire.

11. Procédé selon la revendication 8, 9 ou 10, caractérisé en ce que l'iodure d'alkyle est l'iodure de méthyle.

12. Procédé selon l'une quelconque des revendications précédentes caractérisé en ce qu'on utilise un composé du vanadium dans lequel le vanadium est au degré d'oxydation 4 ou 5.

13. Procédé selon la revendication 12, caractérisé en ce qu'on utilise un composé du vanadium choisi dans le groupe constitué par les oxydes, les halogénures, les oxyhalogénures de vanadium (IV) ou de vanadium (V) et le bis acétylacétonate de vanadyle.

14. Procédé selon l'une quelconque des revendications précédentes caractérisé en ce qu'on utilise le bis acétylacétonate de vanadyle.

15. Procédé selon l'une quelconque des revendications précédentes, caractérisé en ce que le rapport atomique V/Ru est compris entre 0,5 et 500, et de préférence entre 1 et 200.

16. Procédé selon l'une quelconque des revendications précédentes, caractérisé en ce que la température est comprise entre 160 et 250° C.

17. Procédé selon l'une quelconque des revendications précédentes, caractérisé en ce que la pression totale est comprise entre 80 et 350 bars.

18. Procédé selon l'une quelconque des revendications précédentes, caractérisé en ce que le milieu réactionnel initial renferme un acide carboxylique de formule $R'''-COOH$, dans laquelle R''' à la signification donnée pour R, R''' et R pouvant être identiques ou différents.

## Patentansprüche

1. Verfahren zum Hydrocarbonylieren und/oder Carbonylieren von Alkylestern von Carbonsäuren in einem Medium, das zu Beginn mindestens 1 Vol.-% Wasser enthält, unter einem Gesamtdruck von 50

bis 1000 bar und bei einer Temperatur oberhalb 120° C, dadurch gekennzeichnet, daß die Reaktion in Gegenwart von Wasserstoff, Ruthenium, Cobalt, mindestens einem iodhaltigen Promotor und Vanadium durchgeführt wird.

2. Verfahren nach Anspruch 1, dadurch gekennzeichnet, daß der Alkylester einer Carbonsäure der Formel $R-CO-OR'$ entspricht, in der R eine lineare oder verzweigte Alkylgruppe mit 1 bis 16 Kohlenstoffatomen, eine Cycloalkylgruppe mit 3 bis 6 Kohlenstoffatomen, eine Phenylgruppe ($C_6H_5-$), eine Gruppe $C_6H_5-C_xH_{2x}-$ oder eine Gruppe $C_xH_{2x+1}-C_6H_4-$ bedeutet, x eine ganze Zahl von 1 bis 6 ist ($1 \leq x \leq 6$), R' für eine lineare oder verzweigte Alkylgruppe mit 1 bis 5 Kohlenstoffatomen oder für die Gruppe $C_6H_5-C_xH_{2x}-$, in der x die zuvor angegebene Bedeutung hat, steht und R und R' identisch sein können.

3. Verfahren nach Anspruch 2, dadurch gekennzeichnet, daß R' die Methylgruppe bedeutet.

4. Verfahren nach Anspruch 2 oder 3, dadurch gekennzeichnet, daß R eine Alkylgruppe mit höchstens 4 Kohlenstoffatomen oder die Gruppe $C_6H_5-CH_2-$ bedeutet.

5. Verfahren nach Anspruch 1, dadurch gekennzeichnet, daß die Menge Ruthenium 0,5 bis 100 mgAtom, vorzugsweise 1 bis 50 mgAtom je Liter Reaktionsmedium beträgt.

6. Verfahren nach Anspruch 1 oder 5, dadurch gekennzeichnet, daß das Atomverhältnis Co/Ru 0,01 bis 100 und vorzugsweise 0,1 bis 10 beträgt.

7. Verfahren nach einem der vorangehenden Ansprüche, dadurch gekennzeichnet, daß der eingesetzte iodhaltige Promotor ein ionisches Iodid ist, dessen Kation unter den Kationen der Alkalimetalle, der Erdalkalimetalle, der quaternären Ammoniumkationen und der quaternären Phosphoniumkationen ausgewählt ist.

8. Verfahren nach einem der Ansprüche 1 bis 6, dadurch gekennzeichnet, daß der iodhaltige Promotor ein Alkyliodid oder Acyliodid ist.

9. Verfahren nach einem der vorangehenden Ansprüche, dadurch gekennzeichnet, daß das Atomverhältnis I/Ru 0,01 bis 2000, vorzugsweise 0,05 bis 500 beträgt.

10. Verfahren nach Anspruch 8 oder 9, dadurch gekennzeichnet, daß man gleichzeitig ein Alykyliodid und ein ionisches Iodid einsetzt, dessen Kation ausgewählt ist unter den Kationen der Alkalimetalle, der Erdalkalimetalle, der quaternären Ammoniumkationen und der quaternären Phosphoniumkationen.

11. Verfahren nach einem der Ansprüche 8, 9 oder 10, dadurch gekennzeichnet, daß das Alkyliodid das Methyliodid ist.

12. Verfahren nach einem der vorangehenden Ansprüche, dadurch gekennzeichnet, daß man eine Vanadiumverbindung einsetzt, in der das Vanadium in der Oxidationsstufe 4 oder 5 vorhanden ist.

13. Verfahren nach Anspruch 12, dadurch gekennzeichnet, daß man eine Vanadiumverbindung einsetzt, die ausgewählt ist aus der Gruppe bestehend aus den Oxiden, Halogeniden und Oxyhalogeniden von Vanadium (IV) oder Vanadium (V) sowie Vanadyl-bis-acetylacetonat.

14. Verfahren nach einem der vorangehenden Ansprüche, dadurch gekennzeichnet, daß man Vanadyl-bis-acetylacetonat einsetzt.

15. Verfahren nach einem der vorangehenden Ansprüche, dadurch gekennzeichnet, daß das Atomverhältnis V/Ru 0,5 bis 500 und vorzugsweise 1 bis 200 beträgt.

16. Verfahren nach einem der vorangehenden Ansprüche, dadurch gekennzeichnet, daß die Temperatur 160 bis 250° C beträgt.

17. Verfahren nach einem der vorangehenden Ansprüche, dadurch gekennzeichnet, daß der Gesamtdruck 80 bis 350 bar beträgt.

18. Verfahren nach einem der vorangehenden Ansprüche, dadurch gekennzeichnet, daß das Reaktionsmedium zu Beginn eine Carbonsäure der Formel $R'''-COOH$ enthält, in der R''' die für R angegebene Bedeutung hat und R''' und R gleich oder verschieden sind.

**Claims**

1. Process for the hydrocarbonylation and/or carbonylation of alkyl carboxylates in a medium initially containing at least 1% by volume of water, under a total pressure of between 50 and 1000 bars and at a temperature above 120° C, characterised in that the reaction is carried out in the presence of hydrogen, ruthenium, cobalt, at least one iodine-containing promoter and vanadium.

2. Process according to Claim 1, characterised in that the alkyl carboxylate has the formula $R-CO-OR'$, in which R represents a linear or branched alkyl radical having from 1 to 16 carbon atoms, a cycloalkyl radical having from 3 to 6 carbon atoms, a phenyl radical ($C_6H_5-$), a radical $C_6H_5-C_xH_{2x}-$ or a radical $C_xH_{2x+1}-C_6H_4-$, x being an integer between 1 and 6 ($1 \leq x \leq 6$), and R' represents a linear or branched alkyl radical having from 1 to 5 carbon atoms or a radical $C_6H_5-C_xH_{2x}-$, x having the above meaning, it also being possible for R and R' to be identical.

3. Process according to Claim 2, characterised in that R' represents a methyl radical.

4. Process according to Claim 2 or 3, characterised in that R represents an alkyl radical having at most 4 carbon atoms or a radical $C_6H_5-CH_2-$.

5. Process according to Claim 1, characterised in that the amount of ruthenium is between 0.5 and

100 mg atoms, and preferably between 1 and 50 mg atoms, per litre of reaction medium.

6. Process according to claim 1 or 5, characterised in that the atomic ratio of Co/Ru is between 0.01 and 100 and preferably between 0.1 and 10.

7. Process according to any one of the preceding claims, characterised in that the iodine-containing promotor used is an ionic iodide whose cation is chosen from amongst alkali metal cations, alkaline earth metal cations, quaternary ammonium cations and quaternary phosphonium cations.

8. Process according to any one of Claims 1 to 6, characterised in that the iodine-containing promoter. is an alkyl or acyl iodide.

9. Process according to any one of the preceding claims, characterised in that the atomic ratio of I/Ru is between 0.01 and 2000 an preferably between 0.05 and 500.

10. Process according to Claim 8 or 9, characterised in that an alkyl iodide is used in conjunction with an ionic iodide whose cation is chosen from amongst alkali metal cations, alkaline earth metal cations, quaternary ammonium cations and quaternary phosphonium cations.

11. Process according to Claim 8, 9 or 10, characterised in that the alkyl iodide is methyl iodide.

12. Process according to any one of the preceding claims, characterised in that a vanadium compound is used in which the vanadium is an oxidation state of 4 or 5.

13. Process according to Claim 12, characterised in that a vanadium compound is used which is chosen from the group comprising the oxides, halides and oxyhalides of vanadium (IV) or vanadium (V) and vanadyl bis-acetyl-acetonate.

14. Process according to any one of the preceding claims, characterised in that vanadyl bis-acetylacetonate is used.

15. Process according to any one of the preceding claims, characterised in that the atomic ratio of V/Ru ist between 0.5 and 500 and preferably between 1 and 200.

16. Process according to any one of the preceding claims, characterised in that the temperature is between 160 and 250° C.

17. Process according to any one of the preceding claims, characterised in that the total pressure is between 80 and 350 bars.

18. Process according to any one of the preceding claims, characterised in that the initial reaction medium contains a carboxylic acid of the formula R‴—COOH, in which R‴ has the meaning given for R, it being possible for R‴ and R to be identical or different.